(19) **Europäisches Patentamt**
European Patent Office
Office européen des brevets

(11) **EP 3 734 260 B1**

(12) **EUROPÄISCHE PATENTSCHRIFT**

(45) Veröffentlichungstag und Bekanntmachung des Hinweises auf die Patenterteilung:
**01.01.2025 Patentblatt 2025/01**

(21) Anmeldenummer: **20172056.2**

(22) Anmeldetag: **29.04.2020**

(51) Internationale Patentklassifikation (IPC):
**G01N 23/083** (2018.01)  **G01N 33/02** (2006.01)
**G01N 23/087** (2018.01)  **G01N 23/18** (2018.01)

(52) Gemeinsame Patentklassifikation (CPC):
**G01N 33/02; G01N 23/087; G01N 23/18;**
G01N 2223/04; G01N 2223/1016; G01N 2223/3308;
G01N 2223/5015; G01N 2223/618; G01N 2223/633;
G01N 2223/643; G01N 2223/652

(54) **VERFAHREN UND VORRICHTUNG ZUR RÖNTGENINSPEKTION VON PRODUKTEN, INSBESONDERE VON LEBENSMITTELN**

METHOD AND APPARATUS FOR X-RAY INSPECTION OF PRODUCTS, IN PARTICULAR FOOD PRODUCTS

PROCÉDÉ ET DISPOSITIF D'INSPECTION PAR RAYONS X DES PRODUITS, EN PARTICULIER DES DENRÉES ALIMENTAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.05.2019 DE 102019111567**

(43) Veröffentlichungstag der Anmeldung:
**04.11.2020 Patentblatt 2020/45**

(73) Patentinhaber: **WIPOTEC GmbH**
**67657 Kaiserslautern (DE)**

(72) Erfinder:
• **Bur, Christian**
**66113 Saarbrücken (DE)**

• **Wickert, Marco**
**67663 Kaiserslautern (DE)**

(74) Vertreter: **Eder Schieschke & Partner mbB**
**Patentanwälte**
**Elisabethstraße 34**
**80796 München (DE)**

(56) Entgegenhaltungen:
**EP-A1- 2 405 260**  **WO-A1-2017/205914**
**WO-A1-2018/102051**  **DE-A1- 102006 001 681**
**JP-A- 2018 155 754**  **US-A1- 2012 213 331**
**US-A1- 2018 214 113**

**Beschreibung**

Die Erfindung betrifft ein Verfahren und eine Vorrichtung zur Röntgeninspektion von Produkten, insbesondere von Lebensmitteln.

Zur Röntgeninspektion von bewegten Produkten finden üblicherweise Zeilendetektoren Verwendung, die quer zur Bewegungsrichtung der zu untersuchenden Produkte vorgesehen sind. Anstelle einer Bewegung des Produkts kann auch die gesamte Vorrichtung zur Röntgeninspektion oder zumindest die betreffende Röntgenstrahlungsdetektorvorrichtung relativ zu dem zu untersuchenden Produkt bewegt werden. Das zu untersuchende Produkt wird mittels des Zeilendetektors, welcher die von einer oder mehreren Röntgenstrahlungsquellen erzeugte Röntgenstrahlung detektiert, gescannt, und die zeilenweise erzeugten Bilddaten werden in ein Bild des zu untersuchenden Produkts umgesetzt. Zur Erzeugung des Bildes können die Bilddaten in geeigneter Weise verarbeitet werden. Das so erzeugte Bild kann durch Bildverarbeitung inspiziert werden. Insbesondere kann das Bild des zu untersuchenden Produkts daraufhin inspiziert werden, ob ein oder mehrere vorgegebene Produktmerkmale vorliegen bzw. erfüllt sind. Beispielsweise kann ein Lebensmittel, beispielsweise ein Fleischstück, daraufhin untersucht werden, ob sich darin unerwünschte Fremdkörper befinden, wie Knochensplitter, Metallspäne von Verarbeitungsmaschinen, Glassplitter, Kunststoffe, Steine oder dergleichen.

Hierzu ist es bekannt, spektral integrierende Zeilendetektoren (Nicht-Spektraldetektoren) zu verwenden, die praktisch die gesamte Breite des Röntgenstrahlenspektrums der Röntgenstrahlung detektieren, die von der betreffenden Röntgenstrahlungsquelle erzeugt wird. Derartige Zeilendetektoren besitzen eine vergleichsweise hohe Ortsauflösung im Bereich von beispielsweise 0,2 mm über eine Gesamt-Detektorbreite von beispielsweise 200 bis 800 mm oder mehr. Die Zeilendetektoren können dabei in Form von Modulen aufgebaut sein, die sich mit jeweils nur sehr geringen Lücken von nur wenigen Pixeln (beispielsweise ein bis zwei Pixel pro Modulrand) bis zum Erreichen einer gewünschten Scan-Breite koppeln lassen. Mit derartigen Nicht-Spektraldetektoren sind somit auch sehr kleine Fremdkörper bzw. Kontaminationen detektierbar. Dieser Detektortyp benötigt zudem keine Kühlung und ist kostengünstig herstellbar.

Allerdings erzeugen derartige Nicht-Spektraldetektoren infolge der spektralen Integration lediglich Grauwerte. Ein Grauwert ist dabei abhängig von der Dämpfung der Röntgenstrahlung beim Hindurchtreten durch das zu untersuchende Produkt. Die Dämpfung ist dabei wiederum abhängig von der Dicke des Produkts und den Materialeigenschaften.

Dieser Sensortyp eignet sich insbesondere für das Detektieren von kleinsten, stark absorbierenden Fremdkörpern, beispielsweise von Metallsplittern.

Eine Verbesserung des Kontrastes des durch Zeilendetektoren erzeugten Bildes kann durch das Dual-Energy-Verfahren erreicht werden. Dabei werden zwei Zeilendetektoren verwendet, deren Scan-Bilder überlagert werden. Die Zeilendetektoren erfassen dabei unterschiedliche spektrale Bereiche der durch das Produkt hindurchgetretenen Röntgenstrahlung. Dies wird durch das Verwenden wenigstens eines Röntgenstrahlungsfilters erreicht, der im Strahlengang vor einem der Nicht-Spektraldetektoren angeordnet ist. Solche Filter arbeiten jedoch nur als Hochpassfilter und können zudem nicht ausreichend flexibel hinsichtlich der gewünschten Filterkante hergestellt werden. Zusätzlich dämpfen sie auch die zu detektierende Röntgenstrahlung im gewünschten Spektralbereich. Durch das getrennte Erfassen unterschiedlicher Spektralbereiche ist in den Bildsignalen der jeweiligen Zeilendetektoren eine unterschiedliche Information enthalten. Durch eine gewichtete Überlagerung (beispielsweise vorzeichenrichtige Addition der gewichteten Bilddaten) lässt sich ein Gesamtbild erzeugen, welches hinsichtlich der Erkennbarkeit bestimmter Fremdkörpermaterialien einen besseren Kontrast aufweist als ein Single-Energy-Bild. Mit einem festen Röntgenstrahlungsfilter lässt sich der Kontrast jedoch nur für ein oder mehrere bestimmte Materialien verbessern. Daher sind Dual-Energy-Verfahren hinsichtlich ihres Einsatzes wenig flexibel, da das Röntgenstrahlungsfilter, abhängig vom Anwendungsfall, in geeigneter Weise gewählt werden muss.

Zudem überlappen sich meist die Spektralbereiche der Röntgenstrahlung, die von den beiden Zeilendetektoren erfasst werden, so dass in jedem der beiden Bildsignale ein Teil derselben Information enthalten ist. Damit ist keine optimale Kontrastverbesserung erreichbar.

Das Dual-Energy-Verfahren ermöglicht jedoch das Ausblenden von Produktbereichen eines aus lediglich zwei Materialien bestehenden Produkts bei einer geeigneten Kombination der beiden Bildsignale. So kann beispielsweise der Kontrast im Bereich eines Fremdkörpers, d. h. eines ersten Materials, innerhalb des Produkts, d. h. eines zweiten Materials, optimiert werden. Dies gilt jedoch nur für ein im Wesentlichen homogenes Produkt aus einem einzigen Material (oder einer Kombination von Materialien mit sehr ähnlichen Dämpfungseigenschaften für die Röntgenstrahlung), in welchem Fremdkörper aus einem weiteren Material (mit unterschiedlichen Dämpfungseigenschaften für die Röntgenstrahlung) enthalten sind.

Weiterhin wurden in den letzten Jahren spektral auflösende Zeilendetektoren entwickelt, die ebenfalls modulartig gekoppelt werden können. Derartige Spektral-Zeilendetektoren, die ausreichend große Scan-Breiten von 200 bis 800 mm oder mehr ermöglichen, sind derzeit jedoch nur mit einer relativ groben Ortsauflösung, d. h. mit einer verhältnismäßig großen Pixel-Pitch, beispielsweise von 0,8 mm, verfügbar. Derartige Spektral-Zeilendetektoren sind in der Lage, die gesamte spektrale Breite der zu detektierenden Röntgenstrahlung zu erfassen, beispielsweise im Bereich von 20 keV bis 160 keV. Diese Detektoren stellen zur spektralen Auflösung eine Vielzahl, beispielsweise bis zu 256, Energiekanäle bereit. Ein derartiger Spektral-Zeilendetektor ermöglicht es daher, eine der Anzahl von Energiekanälen entsprechende Anzahl von Teilbildern zu erzeugen.

Derartige Zeilendetektoren werden bisher in der Praxis meist zur Erkennung bestimmter Materialien verwendet (z.B. EP 2 588 892 B1), wobei hierzu das für ein Produkt ermittelte Energiespektrum mittels des Energiespektrums eines Hellbildes, d. h. das Spektrum, das von dem Detektor ohne Vorhandensein eines Produkts ermittelt wird, normiert wird. Der natürliche Logarithmus des derart normierten Spektrums entspricht dabei dem Produkt aus dem energieabhängigen Absorptionskoeffizienten für das Produkt multipliziert mit der Dicke des Produkts. Der so für ein unbekanntes Produkt ermittelte spektrale Verlauf des energieabhängigen Absorptionskoeffizienten kann mit den bekannten Produktdaten verglichen werden. Auf diese Weise ist es möglich, das Material eines unbekannten Produkts zu identifizieren.

In der Lebensmittelindustrie besteht häufig das Problem, Produktkontaminationen, d. h. unerwünschte Fremdkörper in einem erwünschten Produkt, zu erkennen. Weisen die zu untersuchenden Produkte Schwankungen in der Dicke auf, so liefern bekannte Verfahren keine sichere Information, ob Schwankungen des Grauwerts in einem Bild, das von einem oder mehreren nicht spektral auflösenden Detektoren erzeugt wird, durch Schwankungen der Dicke des zu untersuchenden Produkts ohne Vorhandensein eines zu detektierenden Fremdkörpers erzeugt werden oder durch das Material eines Fremdkörpers, dass eine abweichende Absorption für die Röntgenstrahlung aufweist.

Zudem liefern bekannte Verfahren und Vorrichtungen zur Röntgeninspektion von Produkten häufig nur Grauwertbilder (dies gilt auch für Dual-Energy-Verfahren) die für eine zuverlässige Auswertung keinen ausreichenden Kontrast aufweisen.

In der US 2018/0214113 A1 wird ein Verfahren und eine Vorrichtung beschrieben, wodurch der Einfluss der energieabhängigen Dämpfung von Materialien auf ein detektiertes Röntgenbild (insbesondere der Einfluss der Strahlaufhärtung (beam hardening)), welches durch die Erfassung der durch ein zu untersuchendes Produkt hindurchgetretenen Röntgenstrahlung einer breitbandigen Röntgenstrahlungsquelle erzeugt wird, kompensiert wird. Hierzu wird ein spektral auflösender Detektor verwendet, dessen Daten der einzelnen Bins mit Korrekturdaten korrigiert werden. Hierdurch ergibt sich ein exakteres bzw. korrekteres, weniger durch den Einfluss der energieabhängigen Dämpfung verfälschtes Bild.

Die US 2012/0213331 A1 beschreibt ein Abtastverfahren mit aus Mikrowellenarrays bestehenden Mikrowellensendern und entsprechend ausgerichteten Mikrowellenempfängern. Das Mikrowellen-Array emittiert kontrolliert gerichtete Mikrowellenstrahlung in Richtung eines zu untersuchenden Objekts. Das zu untersuchende Objekt absorbiert die Strahlung in Abhängigkeit von seinem Metallgehalt. Die Absorption der Mikrowellenstrahlung kann genutzt werden, um eine Messung des Metallgehalts zu erzeugen. Die Messung kann wiederum verwendet werden, um zumindest einen Teil des Volumens und der Form des zu prüfenden Objekts zu berechnen. Der Messwert kann mit einer Vielzahl von vordefinierten Bedrohungen verglichen werden. Dieses System wird vorzugsweise in Kombination mit weiteren Screening-Technologien, wie z.B. der Röntgendurchstrahlungstechnologie, verwendet. Zur Erkennung der Bedrohungen aus den erzeugten Bildern werden u.a. neuronale Netze verwendet.

Schließlich beschreibt die EP 2 405 260 A1 ein Verfahren und eine Vorrichtung zur zerstörungsfreien Röntgeninspektion von Objekten, wobei eine breitbandige Röntgenquelle und ein spektral auflösender Röntgendetektor verwendet werden. Die Bilder der einzelnen Energiekanäle werden zur Darstellung kombiniert, so dass sich ein guter Kontrast ergibt. Die EP 2 405 260 A1 offenbart jedoch nicht, wie die Kombination der einzelnen Bilder erfolgen soll, um einen guten Kontrast zu erreichen. Die Bilder der einzelnen Energiekanäle werden durch statistische Verfahren oder auch Machine-Learning-Verfahren ausgewertet, wobei in jedem Bild Cluster, sogenannte "Blobs", gebildet werden, die Regionen mit derselben Dämpfung bezeichnen. Diese Blobs können in den Bildern der verschiedenen Energiekanäle gegeneinander verschoben sein und in ihrer Größe bzw. Form Differenzen zeigen. Aus den Blobs der verschiedenen Energiekanäle können sogenannte "Superblobs" gebildet werden, die eine Charakteristik darstellen, anhand welcher durch einen Vergleich mit der entsprechenden Charakteristik von bekannten Materialien eine Materialerkennung erfolgen kann.

Ausgehend von diesem Stand der Technik liegt der Erfindung die Aufgabe zugrunde, ein Verfahren zur Röntgeninspektion von Produkten, insbesondere von Lebensmitteln, zu schaffen, das die Erkennung von Fremdkörpern in einem Produkt weiter verbessert. Weiterhin liegt der Erfindung die Aufgabe zugrunde, eine Vorrichtung zur Durchführung des Verfahrens zu schaffen.

Die Erfindung löst diese Aufgabe mit den Merkmalen der Patentansprüche 1 bzw. 9. Weitere Ausführungsformen ergeben sich aus den Unteransprüchen.

Die Erfindung geht von der Erkenntnis aus, dass ein spektral auflösender Röntgenstrahlungsdetektor in vorteilhafter Weise zur Erkennung von Fremdkörpern in einem Produkt verwendet werden kann, wenn die spektralen Daten in geeigneter Weise kombiniert werden. Zudem lassen sich aus den detektierten Daten Informationen über die Dicke von Produkten gewinnen, wenn die Art des Produkts, beispielsweise eines Lebensmittels, wie Fleisch mit Fleisch- und Fettanteil, bekannt ist.

Nach dem erfindungsgemäßen Verfahren wird wenigstens ein Produkttyp definiert, welcher Produkte umfasst, welche aus wenigstens einer ersten und einer zweiten Komponente bestehen, die unterschiedliche Absorptionskoeffizienten für die Röntgenstrahlung aufweisen. Bei Anwendungen in der Lebensmittelindustrie kann es sich hierbei um Fleischstücke handeln, welche die Komponenten Fleisch, Fett und Knochen aufweisen. In diesem Fall ist es meist wünschenswert, das Vorhandensein von Knochen und deren Größe und Lage zu ermitteln. Zudem besteht oftmals der Wunsch, den Fleisch- und Fettanteil zumindest abzuschätzen. In der Lebensmittelindustrie kann es sich bei dem Produkttyp jedoch auch um beliebige andere Produkte handeln, die hinsichtlich ähnlicher Eigenschaften zu untersuchen sind. Beispielsweise kann Joghurt auf das Vorhandensein von Fremdkörpern, wie Stahlabrieb oder Kunststoffpartikel, untersucht werden. Selbstverständlich können nicht nur Stückgüter, sondern auch jegliche Art von Produkten, auch Schüttgüter wie Getreide, Mehl oder dergleichen, in entsprechender Weise untersucht werden.

Das zu untersuchende Produkt, welches dem wenigstens einen Produkttyp zugehört, wird erfindungsgemäß mit einer Röntgenstrahlung mit einer vorgegebenen spektralen Breite durchstrahlt und die durch das Produkt hindurchgetretene Röntgenstrahlung wird mittels des spektral auflösenden Röntgenstrahlungsdetektors detektiert. Der Röntgenstrahlungsdetektor weist eine vorgegebene Anzahl von Pixeln auf, an denen die Röntgenstrahlung spektral aufgelöst detektiert wird. Dabei werden die Röntgenstrahlungsquanten zur spektralen Auflösung, abhängig von deren Energie, einer vorgegebenen Anzahl von Energiekanälen zugeordnet. Der Röntgenstrahlungsdetektor erzeugt auf diese Weise Bilddaten, welche für jedes Pixel Spektralwerte für ausgewählte oder alle Energiekanäle und/oder Summen-Spektralwerte für eine oder mehrere Gruppen von benachbarten Energiekanälen beinhalten.

Nach der Erfindung wird wenigstens eine Abbildungsvorschrift zur Verarbeitung der Bilddaten zu einem Gesamtbild für den wenigstens einen Produkttyp bestimmt, wobei jede Abbildungsvorschrift so ausgebildet ist, dass alle Spektralwerte und Summen-Spektralwerte auf einen Gesamtbildwert eines Bildpunktes des Gesamtbildes abgebildet werden, wobei eine Abbildungsvorschrift jeweils allen Pixeln oder vorbestimmten Gruppen von ein oder mehreren Pixeln zugeordnet wird. Die Variante, wonach eine Abbildungsvorschrift nicht allen Pixeln, sondern nur einer Gruppe von Pixeln zugeordnet wird, ist immer dann von Interesse, wenn der Röntgenstrahlungsdetektor unterschiedliche Empfindlichkeiten aufweist. Meist weist ein derartiger spektral auflösender Röntgenstrahlungsdetektor Kristalle auf, welche zur Detektion der Röntgenstrahlungsquanten dienen. Diese Kristalle können meist nicht mit einer geometrischen Ausdehnung gefertigt werden, die der gewünschten Detektorfläche oder Detektorlänge entspricht, so dass bei einer Kombination mehrerer solcher Kristalle zu einem Röntgenstrahlungsdetektor unterschiedliche Empfindlichkeiten der Kristalle kompensiert werden müssen.

An dieser Stelle sei darauf hingewiesen, dass der Röntgenstrahlungsdetektor als Zeilendetektor oder als Flächendetektor ausgebildet sein kann. Da in der Lebensmittelindustrie häufig die Notwendigkeit besteht, bewegte Produkte über eine Breite von bis zu 800 mm oder mehr zu untersuchen, werden meist Zeilendetektoren verwendet, da Flächendetektoren mit einer derartigen Breite zu teuer wären.

Nach der Erfindung werden die eine oder die mehreren Abbildungsvorschriften so bestimmt, dass in dem Gesamtbild eines Produkts des wenigstens einen Produkttyps eine oder mehrere Komponenten gegenüber einer Referenzkomponente eine Kontraststeigerung erfährt im Vergleich zu einem Grauwertbild, welches durch die einfache Addition aller oder ausgewählter Spektralwerte und aller oder ausgewählter Summen-Spektralwerte zu Gesamtbildpunkten erzeugt würde. Mit anderen Worten, die spektrale Auswertung unter Verwendung einer geeigneten Abbildungsvorschrift ermöglicht eine Verbesserung der Erkennbarkeit von Produktbereichen mit jeweils anderen Materialeigenschaften gegenüber der Verwendung von nicht spektral auflösenden Röntgenstrahlungsdetektoren, die nur ein einfaches Grauwertbild erzeugen.

Nach einer bevorzugten Ausführungsform der Erfindung stellt die wenigstens eine Abbildungsvorschrift einen Klassifikator dar, der die wenigstens zwei Komponenten vorgegebenen Klassen zuordnet. Dabei wird jede Klasse durch einen numerischen Zielwert bezeichnet. Beispielsweise kann in einem Fleischstück, welches aus Fleisch und Knochen besteht, der Knochen als unerwünscht angesehen (und einem unerwünschten Fremdkörper gleichgesetzt) und der Fleischanteil als erwünscht angesehen werden.

Der Klassifikator kann dabei insbesondere ein künstliches neuronales Netz oder eine Support Vector Machine sein.

Erfindungsgemäß wird die wenigstens eine Abbildungsvorschrift so bestimmt, dass jedem Spektralwert oder jedem Summen-Spektralwert ein Abbildungskoeffizient zugeordnet ist, und dass das Gesamtbild des zu untersuchenden Produkts erzeugt wird, indem jeder Spektralwert und jeder Summen-Spektralwert mit dem zugeordneten Abbildungskoeffizienten multipliziert wird und diese Produkte addiert werden. Während die vorstehend geschilderten Varianten ein überwachtes Lernen des Klassifikators voraussetzen, ermöglicht diese Variante auch ein nicht überwachtes Lernen.

Diese Variante ermöglicht darüber hinaus die vorteilhafte Erzeugung eines Dual-Energy-Bildes oder Multiple-Energy-Bildes, wenn diese lineare Abbildungsvorschrift so bestimmt wird, dass aus den Bilddaten ein Dual-Energy-Bild oder Multiple-Energy-Bild erzeugt wird, indem die Spektralwerte von wenigstens zwei Gruppen von ausgewählten benachbarten Energiekanälen oder entsprechende Summen-Spektralwerte mit jeweils einem konstanten Gewichtsfaktor multipliziert werden und diese Produkte addiert werden.

Dabei bietet der spektral auflösende Röntgenstrahlungsdetektor die Möglichkeit, die spektralen Bereiche, die zur Erzeugung des Dual-Energy-Bildes verwendet werden, frei zu wählen. Dabei kann sowohl die Breite der spektralen Bereiche als auch deren Lage beliebig gewählt werden. Zudem kann ein einziger Satz von Bilddaten (d. h. das Bild eines zu untersuchenden Produkts) mit einem einzigen Detektionsvorgang auch mehrmals ausgewertet werden, insbesondere unter Verwendung verschiedener Abbildungsvorschriften, d. h. verschiedener spektraler Bereiche, die zu einem Dual-Energy-Bild kombiniert werden. Somit lassen sich Gesamtbilder erzeugen, die hinsichtlich des Erkennens von bestimmten Materialien, insbesondere von Fremdkörpern aus bestimmten Materialien (beispielsweise Stahl, Kunststoff, Knochen oder dergleichen) optimiert sind.

Nach einer weiteren Ausführungsform kann die wenigstens eine Abbildungsvorschrift so bestimmt werden, dass sich die Gruppen von benachbarten Energiekanälen spektral nicht überlappen, oder dass der Röntgenstrahlungsdetektor so betrieben oder angesteuert wird, dass das Bildsignal nur Spektralwerte für sich nicht überlappende Gruppen von Energiekanälen beinhaltet oder bereits Summen-Spektralwerte für sich nicht überlappende Gruppen von Energiekanälen beinhaltet. Somit lässt sich ein Dual-Energy-Bild erzeugen, welches durch die Kombination von vollkommen disjunkten spektralen Bereichen erzeugt wird. Hierdurch lässt sich gegenüber Dual-Energy-Bildern, die mit herkömmlichen, nicht spektral auflösenden Röntgenstrahlungsdetektoren erzeugt werden, ein verbesserter Kontrast zwischen Bildbereichen erzeugen, die Produktbereichen entsprechen, die in Durchstrahlungsrichtung unterschiedliche Materialkombinationen aufweisen.

Nach der Erfindung wird die wenigstens eine Abbildungsvorschrift mittels eines Machine-Learning-Verfahrens bestimmt, wobei in einem Lernmodus eine Mehrzahl von Trainings-Produkten, die aus jeweils einer der Komponenten bestehen und unterschiedliche Dicken aufweisen, mittels des spektral auflösenden Röntgenstrahlungsdetektors detektiert werden, und wobei zur Bestimmung jeder Abbildungsvorschrift die Spektralwerte oder Summen-Spektralwerte aller oder ausgewählter Pixel oder Gruppen von benachbarten Pixeln, die für die Trainingsprodukte detektiert werden, als Merkmale der Abbildungsvorschrift verwendet werden, und vorgegebene Klassenwerte als Zielwerte der Abbildungsvorschrift verwendet werden, wobei der Klassenwert für alle Trainingsprodukte, die aus derselben Komponente bestehen, identisch ist.

Diese Variante bestimmt die Eigenschaften der wenigstens einen Abbildungsvorschrift derart, dass ein Gesamtbild erzeugt wird, das durch Klassifikation entsteht und demzufolge als Score-Bild aufgefasst werden kann.

Nach einer weiteren Ausführungsform können anstelle von detektierten Spektralwerten oder Summen-Spektralwerten, die für ein Trainingsprodukt aus einer Komponente mit einem bekannten Material gewonnen werden, Simulationsdaten verwendet werden, wobei die Simulationsdaten Produkte aus vorbekannten Werten für den energieabhängigen Massenschwächungskoeffizienten (Absorptionskoeffizient ist gleich dem Massenschwächungskoeffizienten mal der Dichte des Materials) und geeignet gewählten Dicken umfassen.

Diese Vorgehensweise bietet sich insbesondere dann an, wenn die Erkennung von Fremdkörpern in einem Produkt beabsichtigt ist, die aus einem bekannten Material, beispielsweise Stahl oder bestimmten Kunststoffen bestehen, und wenn für dieses Material bereits Daten für den energieabhängigen Absorptionskoeffizienten vorliegen, die entweder in der Inspektionsvorrichtung selbst oder in einer externen Datenbank gespeichert sind. Bei dieser Variante muss daher für das betreffende Material der Absorptionskoeffizient an den Stellen (bzw. in den jeweiligen Bereichen) der Energiekanäle des Röntgenstrahlungsdetektor bestimmt werden. Die betreffenden Werte müssen jeweils mit einer angenommenen Dicke des Materials multipliziert werden. Ein derartiger Wert entspricht dem Wert, den der Röntgenstrahlungsdetektor bei der Durchstrahlung eines derartigen Materials detektieren würde, bezogen auf das jeweilige Hellbild.

Für die Dicke der Trainingsprodukte und auch für die Dicke zur Erzeugung der Simulationsdaten werden vorzugsweise Werte verwendet, die den Bereich abdecken, der bei der Prüfung der Produkte des betreffenden Produkttyps in der Praxis zu erwarten ist.

Nach einer Ausführungsform der Erfindung können mehrere Produkttypen definiert werden, wobei jeder Produkttyp Produkte umfasst, welche aus jeweils derselben ersten und wenigstens einer zweiten Komponente bestehen, die unterschiedliche Absorptionskoeffizienten für die Röntgenstrahlung aufweisen. So lassen sich unter Verwendung derselben Bilddaten eines zu untersuchenden Produkts mehrere Gesamtbilder erzeugen, wobei zur Erzeugung jedes Gesamtbildes eine Abbildungsvorschrift für einen jeweils anderen Produkttyp verwendet wird.

Die Erfindung wird nachstehend unter Bezugnahme auf die Zeichnung näher erläutert. In der Zeichnung zeigen:

Fig. 1    eine schematische Darstellung einer Ausführungsform einer Vorrichtung zur Inspektion von Produkten mit einem spektral auflösenden Zeilendetektor;

Fig. 2    ein beispielhaftes Energiespektrum, das von einem Pixel eines spektral auflösenden Zeilendetektors erzeugt wurde;

Fig. 3    ein Gewichtungsprofil zur Gewichtung der Energiekanäle eines Energiespektrums gem. Fig. 2 zur Erzeugung eines Dual-Energy-Bildes;

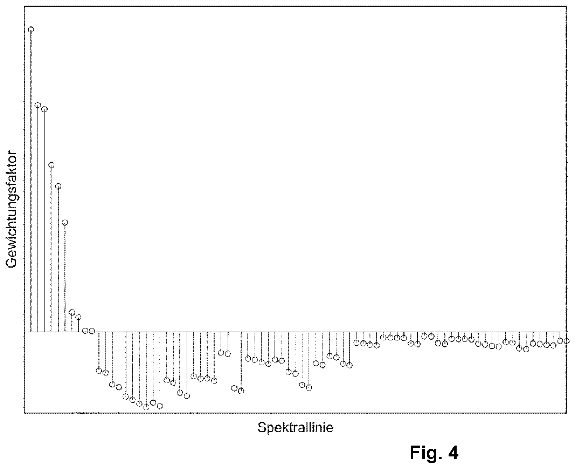

Fig. 4    ein Gewichtungsprofil zur Gewichtung der Energiekanäle eines Energiespektrums gem. Fig. 2 zur Erzeugung eines Score-Bildes;

Fig. 5     ein Dual-Energy-Grauwertbild von überlappenden Produkten und Fremdkörpern aus einem ersten und einem zweiten Material (Fig. 5a) sowie ein erstes Score-Bild, welches zur Sichtbarmachung der Fremdkörper aus dem ersten Material optimiert ist (Fig. 5b), und ein zweites Score-Bild, welches zur Sichtbarmachung der Fremdkörper aus dem zweiten Material optimiert ist (Fig. 5c); und

Fig. 6     eine Tabelle zur Erläuterung von Auswerteverfahrens für die Bilddaten.

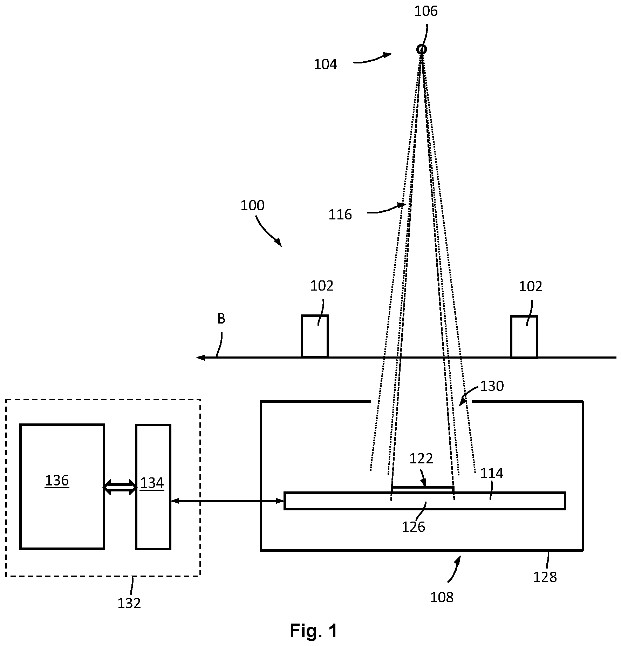

Fig. 1 zeigt eine schematische Darstellung einer ersten Ausführungsform einer Vorrichtung 100 zur Röntgeninspektion von Produkten 102, insbesondere von Lebensmitteln, mit einer Strahlungserzeugungsvorrichtung 104 mit wenigstens einer Röntgenstrahlungsquelle 106 mit einer Röntgenstrahlungsdetektorvorrichtung 108 und mit einem spektral auflösenden Röntgenstrahlungsdetektor 114, welcher als Zeilendetektor ausgebildet ist.

Die Röntgenstrahlungsquelle 106 erzeugt einen fächerförmigen Röntgenstrahl 116, welcher eine Mittelebene aufweist, die senkrecht zu einer Bewegungsrichtung B steht, in welcher die zu untersuchenden Produkte 102 durch den Röntgenstrahl 116 bewegt werden. Der Röntgenstrahl 116 weist in der Bewegungsebene E einen Öffnungswinkel auf, der so ausgebildet ist, dass das zu untersuchende Produkt 102 in seiner gesamten Breite von dem Röntgenstrahl 116 durchstrahlt wird. Zur Bewegung des Produkts 102 kann eine Fördervorrichtung (nicht dargestellt), beispielsweise ein Transportband, vorgesehen sein.

Der Zeilendetektor 114 umfasst eine Detektorzeile 122, welche eine diskrete Ortsauflösung, d. h. eine Pixel-Pitch, von beispielsweise 0,8 mm aufweisen kann. Die Detektorzeile 122 ist dabei in etwa mittig auf einem Träger 126 vorgesehen, der auch Kühlkörper und andere Komponenten tragen kann. Die Kühlkörper können auch den Träger 126 bilden.

Der Zeilendetektor 114 kann, wie in Fig. 1 gezeigt, in einem Gehäuse 128 vorgesehen sein, welches als Strahlenschutzgehäuse ausgebildet sein kann. Das Gehäuse 128 weist an seiner Oberseite, d. h. der der Röntgenstrahlungsquelle 106 zugewandten Seite, eine Öffnung 130 auf, welche das Eindringen des Röntgenstrahls 116 in das Gehäuse in Richtung auf die Pixelzeile 122 des Zeilendetektors 114 ermöglicht.

Anstelle eines einzigen spektral auflösenden Zeilendetektors 114 können auch zwei oder mehrere spektral auflösende Zeilendetektoren vorgesehen sein. Dies kann dann von Vorteil sein, wenn die nicht spektral auflösenden Zeilendetektoren jeweils zur Erfassung einer unterschiedlichen maximalen spektralen Breite ausgebildet sind. Beispielsweise kann einer der spektral auflösenden Zeilendetektoren eine spektrale Breite von maximal 20 keV bis 160 keV mit einer spektralen Auflösung von 256 Energiekanälen aufweisen und ein weiterer spektral auflösender Zeilendetektor eine spektrale Breite von maximal 20 keV bis 80 keV, ebenfalls bei einer Auflösung von 256 Energiekanälen. Der weitere spektral auflösende Zeilendetektor weist somit eine doppelt so hohe spektrale Auflösung auf wie der erste spektral auflösende Zeilendetektor.

Der Zeilendetektor 114 erzeugt ein Bilddatensignal, welches einer Auswerte- und Steuereinheit 132 zugeführt ist. Die Auswerte- und Steuereinheit 132 kann eine Datenerfassungseinheit 134 und eine Bildverarbeitungseinheit 136 aufweisen. Der Datenerfassungseinheit 134 ist das Bilddatensignal des Zeilendetektors 114 zugeführt. Die Bildverarbeitungseinheit 136 ist zur weiteren Verarbeitung und Analyse der Bilddaten ausgebildet. Die Datenerfassungseinheit 134 kann auch so ausgebildet sein, dass diese den Zeilendetektor 114 in geeigneter Weise ansteuert, insbesondere hinsichtlich der Abtastzeitpunkte. Hierzu kann die Datenerfassungseinheit 134 dem Zeilendetektor ein Taktsignal zuführen, wobei die Bilddatenerfassung durch den Zeilendetektor synchronisiert mit dem Taktsignal erfolgen kann.

Die Bildverarbeitungseinheit 136 kann die vom Zeilendetektor 114 erfassten Bilddaten in der nachstehenden Art und Weise verarbeiten.

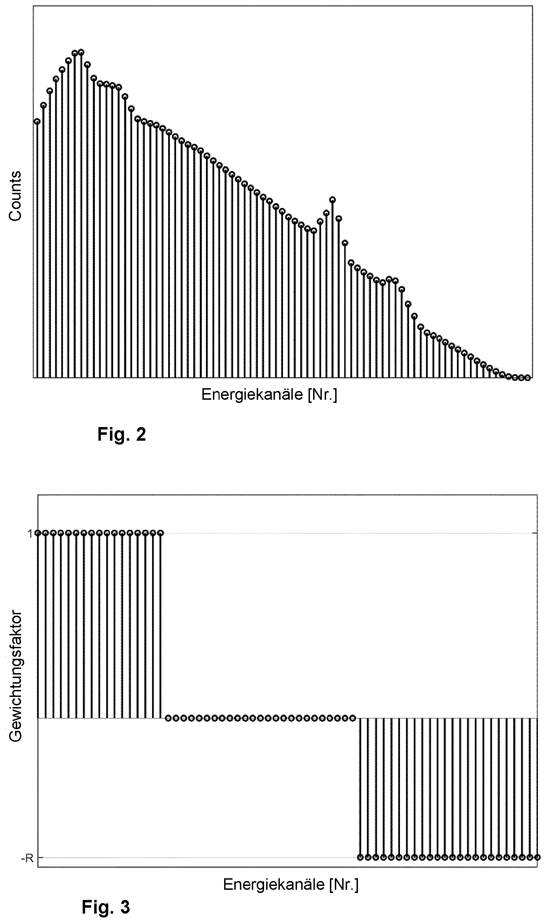

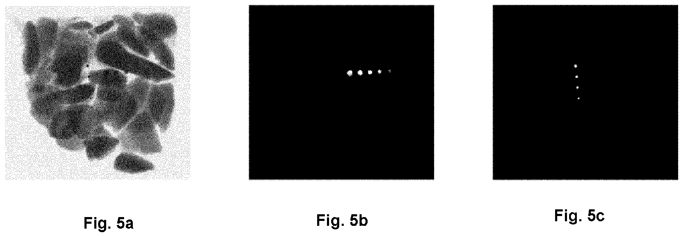

Fig. 2 zeigt beispielhaft die Bilddaten, die von dem spektral auflösenden Zeilendetektor 114 für ein bestimmtes Pixel geliefert werden. Hierbei ist der gesamte erfasste Energiebereich, beispielsweise von 20 keV bis 80 keV, in eine bestimmte Anzahl von Energiekanälen eingeteilt, wobei jeder Energiekanal eine bestimmte (üblicherweise konstante) spektrale Breite aufweist, die sich aus der Breite des gesamten erfassten spektralen Bereichs geteilt durch die Anzahl von Energiekanälen, beispielsweise 128 oder 256 Energiekanäle, ergibt. Der Zeilendetektor 114 liefert für jeden Energiekanal einen Spektralwert, der in Fig. 5 mit "Counts" bezeichnet ist, da ein derartiger spektral auflösender Zeilendetektor in der Regel einzelne Photonen zählt, und die registrierten Photonen, abhängig von deren Energie, einem bestimmten Energiekanal zuordnet. Der in Fig. 5 dargestellte Verlauf entspricht einem typischen Hellbild, welches von dem Zeilendetektor 114 ohne das Vorhandensein eines Produkts im Strahlengang des Röntgenstrahls 116 erzeugt wird. Dieses Spektrum wird in charakteristischer Weise verändert, wenn ein Produkt aus einem bestimmten Material oder einer bestimmten Materialkombination im Strahlengang der Röntgenstrahlung liegt, die durch das Pixel detektiert wird.

Die Spektralwerte werden mit dem Bilddatensignal der Auswerte- und Steuereinheit 136 als Bilddaten übermittelt. Die Auswerte- und Steuereinheit 136 kann diese Bilddaten auf verschiedenartige Weise auswerten.

Beispielsweise kann die Fähigkeit einer spektralen Auflösung des Zeilendetektors 114 genutzt werden, um ein Dual-Energy-Bild zu erzeugen. Hierzu kann die Auswerte- und Steuereinheit 136 pixelweise eine beliebige Gewichtung der Spektralwerte vornehmen. Eine solche Gewichtung kann dadurch bewirkt werden, dass jedem einzelnen Energiekanal ein Faktor zugeordnet wird, der mit dem jeweiligen Spektralwert multipliziert wird. Hierdurch kann auch eine scharfe Beschränkung des Spektrums erreicht werden, wenn ausgewählten Energiekanälen der Faktor Null zugeordnet wird.

Fig. 3 zeigt ein Gewichtungsprofil, welches für jeden Energiekanal einen separaten Gewichtungsfaktor aufweist. Dieses Profil sieht für eine Anzahl von Energiekanälen am unteren Bereich des gesamten Spektrums und am oberen Bereichs des Spektrums jeweils eine konstante Gewichtung vor, beispielsweise mit einem Faktor 1 im unteren Spektralbereich und mit einem Faktor -R im oberen Spektralbereich. Alle anderen Energiekanäle werden mit dem Faktor 0 gewichtet. Die so gewichteten Spektralwerte der Energiekanäle können zur Erzeugung eines Summen-Spektralwerts addiert werden, wodurch sich ein Dual-Energy-Bild ergibt.

Wie bereits vorstehend erläutert, kann der spektral auflösende Zeilendetektor 114 auch so ausgebildet sein, dass dieser eine Vorauswahl trifft, welche Energiekanäle im Rahmen eines Bilddatensignals zur Auswerte- und Steuereinheit 136 übermittelt werden. Beispielsweise kann der Zeilendetektor 114 manuell oder von der Auswerte- und Steuereinheit 136 so eingestellt werden, dass er nur bestimmte Energiekanäle als Bilddatensignal ausgibt. Der Zeilendetektor 114 kann auch so ausgebildet sein, dass er die ausgewählten Energiekanäle bereits integriert ausgibt, d. h. die Spektralwerte der ausgewählten Energiekanäle aufsummiert. In diesem Fall ergibt sich für die Auswerte- und Steuereinheit 136 ein geringerer Aufwand für die Verarbeitung der Bilddaten des Zeilendetektors 114.

Somit ermöglicht der nicht spektral auflösende Zeilendetektor 114 die Erzeugung eines Dual-Energy-Bildes unter Verwendung eines flexiblen Spektrums. Dieses kann durch die einfache Auswertung des Bilddatensignals des Zeilendetektors 114 festgelegt werden, oder der Zeilendetektor 114 wird so angesteuert, dass er bereits entsprechende spektral beschränkte Bilddaten bzw. sogar Summen-Spektralwerte liefert (siehe oben).

Das Spektrum des spektral auflösenden Zeilendetektors 114 kann dabei so variiert werden, dass bestimmte Produktmerkmale eines zu untersuchenden Produkts in dem Dual-Energy-Bild besser, beispielsweise mit einem höheren Kontrast, erkennbar sind.

Die Auswertung der mittels eines einzigen Scans gewonnenen Bilddaten kann auch so erfolgen, dass mehrere Auswertungen durchgeführt werden. Insbesondere können unterschiedliche Dual-Energy-Bilder unter Verwendung verschieden gewichteter Bilddaten des spektral auflösenden Zeilendetektors 114 erzeugt werden. Beispielsweise kann das Spektrum der Bilddaten des Zeilendetektors 114 in einer Auswertung so gewählt werden (beispielsweise durch eine entsprechende Gewichtung), dass Fremdkörper aus einem bestimmten Material, beispielsweise Stahl, mit hohem Kontrast erkennbar sind. In einer weiteren Auswertung kann das Spektrum der Bilddaten des Zeilendetektors 114 anders gewählt werden, beispielsweise um ein Dual-Energy-Bild zu erzeugen, in dem Fremdkörper aus einem anderen Material, beispielsweise Polyethylen, mit hohem Kontrast zu erkennen sind.

Im Folgenden wird erläutert, wie die vollständigen Informationen, die in den spektralen Bilddaten enthalten sind, durch eine vorteilhafte Bildauswertung verwendet werden können.

Hierzu wird zunächst eine Einlernphase durchlaufen, wozu die Auswerte- und Steuereinheit in einen Einlernmodus überführt werden kann. In der Einlernphase wird wenigstens eine Abbildungsvorschrift bestimmt, die alle oder ausgewählte Spektralwerte und Summen-Spektralwerte auf einen Gesamtbildwert eines Bildpunktes des Gesamtbildes abbildet, oder die alle oder ausgewählte Spektralwerte und Summen-Spektralwerte auf einen Gesamtbildwert eines Bildpunktes des Gesamtbildes abbildet, welcher einen Wert für die Gesamtdicke einer Komponente des durchstrahlten Produkts, in der Durchstrahlungsrichtung gesehen, darstellt.

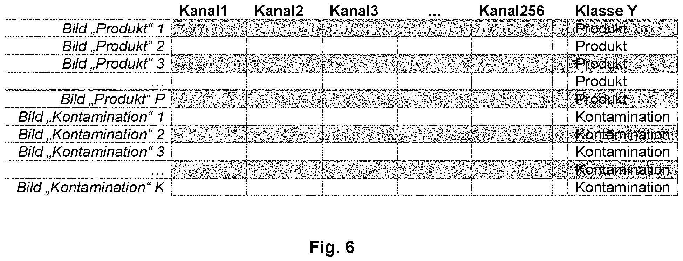

Beispielsweise können in der Einlernphase die einzelnen Pixel der aufgenommen Produktbilder mit ihren zugehörigen 256 Energiekanälen in Form einer Tabelle, wie in Fig. 6 dargestellt, angeordnet werden. Dabei bilden die Pixel die Zeilen, sogenannte Beobachtungen. Die Energiekanäle jedes Pixels bilden die Spalten und werden als Merkmale bezeichnet.

Jeder Zeile der Tabelle, und damit jedem Pixel, wird ein Klassenwert Y (Zielwert) zugeordnet. Dieser kann - abhängig von der Anwendung - im Fall einer Kontrastoptimierung entweder ein diskreter Klassenname oder eine Klassenidentifikationsnummer sein, oder im Fall einer Schichtdickenbestimmung ein Schichtdickenwert, beispielsweise die Schichtdicke in mm. Damit sind in der Einlernphase die Merkmale und die Klassenwerte bekannt und es gilt eine Abbildungsvorschrift zu bestimmen.

Im Fall einer Kontrastoptimierung für zu untersuchende Produkte, die eine Kontamination mit Fremdkörpern aufweisen können, können für den Einlernprozess mehrere Bilder einer ersten Komponente (nicht kontaminiertes Produkt), vorzugsweise mit verschiedenen Dicken aufgenommen werden. Den Pixeln dieser Bilder wird ein Klassenwert, z.B. "Produkt", zugeordnet. Darüber hinaus werden Bilder weiterer Komponenten aufgenommen, die einen von der ersten Komponente verschiedenen Absorptionskoeffizienten besitzen. Auch diesen Pixeln wird ein Klassenwert, z.B. "Kontamination", zugewiesen. Beide Datensätze werden in einer Tabelle, wie in Fig. 6 dargestellt, zusammengefasst, welche demzufolge mindestens zwei verschiedene Klassenwerte Y beinhaltet.

Nach einer weiteren Ausführungsform kann für das Einlernen nur die Komponente 1 mittels des Röntgenstrahlendetektors detektiert werden. Für die wenigstens eine weitere Komponente können Mess- oder auch Simulationsdaten auf der Maschine hinterlegt sein und zur Erstellung einer Tabelle gemäß Fig. 6 genutzt werden.

Simulationsdaten der energieabhängigen Massenschwächungskoeffizienten (Massenschwächungskoeffizienten = Absorptionskoeffizient dividiert durch Dichte) aller Elemente des Periodensystems sind in Datenbanken frei verfügbar. Aus diesen kann der energieabhängige Massenschwächungskoeffizient von Molekülen, und damit auch von Materialkombinationen, bestimmt werden.

Diese Daten können auf der Maschine vorgehalten und zusammen mit realen Messdaten zur Bestimmung der Abbildungsvorschrift genutzt werden. Für das Einlernen muss in solchen Fällen lediglich das nichtkontaminierte Produkt (Komponente 1) mit dem Röntgenstrahlungsdetektor detektiert (gescannt) werden und potentielle Kontaminationen (d. h. weitere Komponenten) werden über Simulationsdaten der Merkmalstabelle als weitere Zeilen zugeführt. Hierzu wird der Massenschwächungskoeffizient einer potentiellen Kontamination (typischerweise Eisen, Edelstahl, Kunststoffe, Glas) mit einer durchschnittlichen Dichte der Kontamination bei Betriebstemperatur sowie mit mehreren realistischen Dicken multipliziert, um so künstlich die Absorptionseigenschaften der Kontaminationen zu generieren. Vorteilhaft an dieser Vorgehensweise ist, dass ein Anwender lediglich eine ausreichende Anzahl von Produkten (vorzugsweise mit einem Querschnitt deren Eigenschaften) aus seiner Produktionslinie für den Einlernprozess scannen, d. h. mittels des Röntgenstrahlungsdetektors entsprechende Spektralwerte erzeugen muss, da die erforderlichen Fremdkörperdaten (Kontaminationsdaten) bereits auf der Maschine vorliegen oder über Simulationsdaten erzeugt werden.

Nach dem Gewinnen der Einlerndaten wird eine Abbildungsvorschrift gesucht, die - im Fall der Kontrastverbesserung - die Merkmale anhand ihres Klassenwertes Y in eine andere Darstellungsform, insbesondere ein sogenanntes Score-Bild, transformiert. Das Score-Bild weist verbesserte Eigenschaften gegenüber den Rohbildern auf. Die Abbildungsvorschrift kann jedoch auch direkt eine Klassifikation durchführen.

Eine verbesserte Eigenschaft in diesem Zusammenhang ist beispielsweise der Abstand der Gesamtbildwerte einer ersten Komponente (z.B. Joghurt) gegenüber mindestens einer weiteren Komponente (z.B. Glas-Kontamination), im Rahmen dieser Beschreibung auch als Kontrast bezeichnet. Ziel ist es, die weitere (Kontaminations-) Komponente im transformierten Bild deutlicher, d.h. kontraststärker, gegenüber der ersten Komponente darzustellen. Im Idealfall wird eine der Komponenten (meist die erste Komponente) ausgeblendet, so dass nur noch alle weiteren Komponenten sichtbar sind.

Über den Klassenwert in der Tabelle gemäß Fig. 6 kann gesteuert werden, welche Komponenten ausgeblendet werden sollen, da die Abbildungsvorschrift basierend auf den verschiedenen Klassenwerten eine Maximierung des Klassenabstandes vornimmt. Mehrere verschiedene Komponenten können zu einem Klassenwert zusammengefasst werden.

Nach einer speziellen Variante wird hierzu jedem Energiekanal ein Abbildungskoeffizient (Gewichtungsfaktor) c zugeordnet. Die so gebildeten Produkte werden aufsummiert, d. h. es wird damit eine Linearkombination der Merkmale gebildet.

$$Y_1 = c_1 \cdot Kanal1_1 + c_2 \cdot Kanal2_1 + ... + c_{256} \cdot Kanal256_1$$
$$Y_2 = c_1 \cdot Kanal1_2 + c_2 \cdot Kanal2_2 + ... + c_{256} \cdot Kanal256_2$$
$$\vdots$$
$$Y_B = c_1 \cdot Kanal1_B + c_2 \cdot Kanal2_B + ... + c_{256} \cdot Kanal256_B$$

Zur Bestimmung der Abbildungskoeffizienten c können Verfahren aus dem Bereich der multivariaten Statistik angewendet werden, bspw. eine Korrelations- oder Diskriminanzanalyse.

In der Einlernphase werden Abbildungskoeffizienten c bestimmt, welche in der anschließenden Produktionsphase zur Berechnung der Score-Bilder (Ergebniswerte) zur Anwendung kommen. In der Produktionsphase werden Bilder aufgenommen und die so erzeugten Merkmale (d. h. Spektralwerte der Energiekanäle) werden mit den Abbildungskoeffizienten c verrechnet, wodurch neue Klassenwerte Y (Ergebniswerte) bestimmt werden, die sogenannten Scores. In dem Score-Bild ist der Kontrast, also der Abstand der Gesamtbildwerte, zwischen den einzelnen Komponenten gegenüber dem Rohbild deutlich erhöht. Eine nachgeschaltete Bildverarbeitung kann in dem Score-Bild deutlich einfacher (sicherer und mit höherer Empfindlichkeit) die einzelnen Komponenten unterscheiden. Dies ist insbesondere für eine Fremdkörperdetektion vorteilhaft, da die Fehldetektion reduziert wird und auch kleine oder schwach absorbierende Kontaminationen zuverlässig erkannt werden.

Fig. 5 zeigt einen Vergleich eines in Fig. 5a dargestellten Grauwertbildes, bei dem es sich um ein Dual-Energy-Bild handeln kann, mit zwei Score-Bildern, welche zwei verschiedenartige Kontaminationen (aus unterschiedlichen Materialien), die auch in dem Graustufenbild schwach erkennbar sind, deutlich erkennen lassen (Fig. 5b und Fig. 5c). In diesem Fall wurden also zwei verschiedene Abbildungsvorschriften parallel zum Einsatz gebracht, um die verschiedenartigen Kontaminationen zu identifizieren.

In einer weiteren Ausführungsform wird die Abbildungsvorschrift als Klassifikator aufgefasst, welcher in der Produktionsphase jedem Pixel eine Klasse zuordnet. Die Einlernphase dient nun dazu den Klassifikator zu trainieren. Hierzu wird - analog zu der oben beschriebenen Transformation - die Tabelle gemäß Fig. 6 genutzt. Als Klassifikator kann beispielsweise eine Support Vector Machine (SVM) genutzt werden. Diese sucht eine Hyperebene, die beide Klassen, d. h. die erste und eine weitere Komponente, welche durch die Merkmale (Spektralwerte der Energiekanäle) beschrieben werden, bestmöglich voneinander trennt. Eine Hyperebene ist im einfachsten Fall eine Gerade, die die beiden Gruppen voneinander trennt. Sind die Klassen nicht linear trennbar, werden die Merkmale in einen höher dimensionalen Raum überführt, in welchem sie linear trennbar sind. Diese Trennlinie in einem höher dimensionalen Raum wird als Hyperebene bezeichnet.

Die Abbildungsvorschrift ist im Falle eines Klassifikators keine Transformation der Energiekanäle in einen neuen Bildwert (Score), welcher weiter analysiert werden muss, im eigentlichen Sinne, sondern weist jedem Pixel (Beobachtung) direkt eine Klassenzugehörigkeit (Ergebniswert) zu.

In einer weiteren Ausführungsform wird ein künstliches Neuronales Netz (ANN) genutzt. Dieses kann entweder die Abbildungskoeffizienten als Vorfaktoren der Spektralwerte und/oder Summenspektralwerte genutzt werden - und damit eine Transformation wie die oben skizzierte Linearkombination durchführen -, oder das ANN stellt direkt den Klassifikator dar, welcher in der Produktionsphase jedem Pixel (Beobachtung) einen Klassenwert zuweist.

In einer weiteren Ausführungsform wird eine Transformation der Energiekanäle in ein Score-Bild mit Hilfe der Faktorenanalyse, insbesondere auch der Hauptkomponentenanalyse, durchgeführt. Diese nimmt eine Transformation der Merkmale (Energiekanäle) anhand der, in dem Datensatz enthaltenen Varianz, vor, ohne Berücksichtigung der im Einlernprozess bekannten Klassenwerte.

Neben den einzelnen Spektral- und/oder Summen-Spektralwerten der Energiekanäle, die als Merkmale aufgefasst werden, sind auch beliebige Kombinationen der Energiekanäle untereinander und/oder mit sich selbst denkbar. So kann es von Vorteil sein, dass beispielsweise Energiekanäle quadriert werden und damit 256 weitere Merkmale generiert werden. Auch eine Kombination von Merkmalen untereinander, sogenannter Mischterme, ist denkbar. In obiger Gleichung treten die Kanäle nicht ausschließlich linear auf, sondern in beliebiger Ordnung. Die Abbildungsvorschrift selbst ist weiterhin linear, wobei die Eingangsdaten vorverarbeitet werden. Dies ist insbesondere dann von Vorteil, wenn die Klassen nicht linear trennbar sind, aber eine Transformation mittels einer Linearkombination, wie oben skizziert, angestrebt wird.

Im Fall der Schichtdickenbestimmung erfolgt der Einlernprozess häufig mit (mindestens) zwei Referenzmaterialen. Dabei variiert die Schichtdicke einer ersten Komponente bei gleichbleibender Schichtdicke einer weiteren Komponente, und anschließend umgekehrt. Für jede Konfiguration an Referenzmaterialien sind die Schichtdicken der beiden Komponenten bekannt und werden als Klassenwert Y in der Tabelle gemäß Fig. 6 verwendet. In einer vorteilhaften Ausführungsform wird für jede in dem Produkt enthaltene Komponente ein separates Modell bestimmt. Hierzu wird für jede Komponente eine eigene Tabelle, wie in Fig. 6 dargestellt, aufgestellt. Aus dem Verhältnis der Schichtdicken lässt sich ein Verhältnis der ersten Komponente zu der weiteren Komponente bestimmen. Ein Praxisbeispiel ist hierbei die Bestimmung des Fettanteils in Fleisch, der oftmals als chemical lean value (CL-Wert) angegeben wird. Nach dem Erstellen der Merkmalstabelle gemäß Fig. 6 wird wiederum eine Abbildungsvorschrift gesucht, die die Spektralwerte und/oder Summenspektralwerte der Energiekanäle einer Schichtdicke mindestens einer der Komponenten zuweist.

Erfindungsgemäß wird eine Linearkombination der Spektral- und/oder Summenspektralwerte (auch Produkte aus Energiekanälen sind möglich) gebildet, also jedem Merkmal ein Abbildungskoeffizient c zugewiesen.

$$Y_1 = c_1 \cdot Kanal1_1 + c_2 \cdot Kanal2_1 + ... + c_{256} \cdot Kanal256_1$$

$$Y_2 = c_1 \cdot Kanal1_2 + c_2 \cdot Kanal2_2 + ... + c_{256} \cdot Kanal256_2$$

$$Y_B = c_1 \cdot Kanal1_B + c_2 \cdot Kanal2_B + ... + c_{256} \cdot Kanal256_B$$

Y stellt in der vorstehenden Gleichung die Schichtdicke einer Komponente dar.

Die Abbildungsvorschrift zur Bestimmung von Schichtdicken wird als Regressionsproblem aufgefasst. Die Abbildungskoeffizienten c selbst werden in einer ersten Ausführungsform mit Hilfe einer multiplen linearen Regression (O-PLS, ordinary partial least squares) bestimmt.

In der Produktionsphase werden die gefundenen Koeffizienten angewendet, um damit eine Schichtdicke Y vorherzusagen.

In einer weiteren Ausführungsform wird ein künstliches, neuronales Netz (ANN) genutzt, um die Schichtdicke Y basierend auf den Spektral- und/oder Summenspektralwerten zu schätzen.

Weitere gängige Verfahren zur Lösung dieses Regressionsproblems sind Support Vector Regression (SVR) oder Gaussian Process Regression (GPR).

In einer weiteren Ausführungsform wird anstelle der Schichtdicken der Einzelkomponenten direkt das (Massen-)verhältnis der Komponenten im Produkt bestimmt. In der Tabelle gemäß Fig. 6 ist der Klassenwert nun das (Massen-)Verhältnis der Komponenten und nicht mehr die Schichtdicke einzelner Komponenten, aus denen das (Massen-)verhältnis bestimmt wird.

Bezugszeichenliste

| | |
|---|---|
| 100 | Vorrichtung zur Röntgeninspektion |
| 102 | Produkt |
| 104 | Strahlungserzeugungsvorrichtung |
| 106 | Röntgenstrahlungsquelle |
| 108 | Röntgenstrahlungsdetektorvorrichtung |
| 114 | spektral auflösender Zeilendetektor |
| 116 | fächerförmiger Röntgenstrahl |
| 118 | Pixelzeile |
| 120 | Pixelzeile |
| 122 | Pixelzeile |
| 124 | Träger |
| 126 | Träger |
| 128 | Gehäuse |
| 130 | Öffnung |
| 132 | Auswerte- und Steuereinheit |
| 134 | Datenerfassungseinheit |
| 136 | Bildverarbeitungseinheit |
| 200 | Röntgenstrahlungsdetektorvorrichtung |
| B | Bewegungsrichtung |

**Patentansprüche**

1. Verfahren zur Röntgeninspektion von Produkten eines vorgegebenen Produkttyps, insbesondere von Lebensmitteln,

(a) bei dem wenigstens ein Produkttyp definiert wird, welcher Produkte (102) umfasst, welche aus wenigstens einer ersten und einer zweiten Komponente bestehen, die unterschiedliche Absorptionskoeffizienten für die Röntgenstrahlung aufweisen,

(b) bei dem ein zu untersuchendes Produkt (102), welches dem wenigstens einen Produkttyp zugehört, mit einer Röntgenstrahlung mit einer vorgegebenen spektralen Breite durchstrahlt wird,

(c) bei dem die durch das Produkt (102) hindurchgetretene Röntgenstrahlung mittels eines spektral auflösenden Röntgenstrahlungsdetektors (200) detektiert wird, welcher eine vorgegebene Anzahl von Pixeln aufweist und die Röntgenstrahlung an jedem Pixel diskret spektral aufgelöst detektiert, wobei der Röntgenstrahlungsdetektor (200) die Röntgenstrahlungsquanten zur spektralen Auflösung, abhängig von deren Energie, einer vorgegebenen Anzahl von Energiekanälen zuordnet und Bilddaten erzeugt, welche für jedes Pixel Spektralwerte für ausgewählte oder alle Energiekanäle und/oder Summen-Spektralwerte für eine oder mehrere Gruppen von benachbarten Energiekanälen beinhalten, und

(d) bei dem zur Verarbeitung der Bilddaten zu einem Gesamtbild für den wenigstens einen Produkttyp wenigstens eine Abbildungsvorschrift bestimmt wird, wobei jede Abbildungsvorschrift so ausgebildet ist, dass alle oder ausgewählte Spektralwerte und Summen-Spektralwerte auf einen Gesamtbildwert eines Bildpunktes des Gesamtbildes abgebildet werden, wobei eine Abbildungsvorschrift jeweils allen Pixeln oder vorbestimmten Gruppen von ein oder mehreren Pixeln zugeordnet wird, und

(e) wobei die eine oder mehreren Abbildungsvorschriften so bestimmt werden, dass in dem Gesamtbild eines Produkts (102) des wenigstens einen Produkttyps eine Verbesserung der Erkennbarkeit von Produktbereichen mit jeweils anderen Materialeigenschaften gegenüber der Verwendung von nicht spektral auflösenden Röntgenstrahlungsdetektoren erreicht wird, die nur ein einfaches Grauwertbild erzeugen,

**dadurch gekennzeichnet,**

(f) **dass** die wenigstens eine Abbildungsvorschrift als Linearkombination bestimmt wird, indem jedem Spektralwert oder jedem Summen-Spektralwert ein Abbildungskoeffizient zugeordnet ist, und dass das Gesamtbild des zu untersuchenden Produkts (102) erzeugt wird, indem jeder Spektralwert und jeder Summen-Spektralwert mit dem zugeordneten Abbildungskoeffizienten multipliziert wird und diese Produkte addiert werden, und

(g) **dass** die wenigstens eine Abbildungsvorschrift für den wenigstens einen Produkttyp zur Verbesserung des Kontrastes mittels eines Machine-Learning-Verfahrens bestimmt wird,

(i) wobei in einem Lernmodus eine Mehrzahl von Trainings-Produkten, die aus jeweils einer der Komponenten bestehen und unterschiedliche Dicken aufweisen, mittels des spektral auflösenden Röntgenstrahlungsdetektors (200) detektiert werden,
(ii) wobei zur Bestimmung jeder Abbildungsvorschrift

(1) die Spektralwerte oder Summen-Spektralwerte aller oder ausgewählter Pixel oder Gruppen von benachbarten Pixeln, die für die Trainingsprodukte detektiert werden, als Merkmale der Abbildungsvorschrift verwendet werden,
(2) vorgegebene Klassenwerte als Zielwerte der Abbildungsvorschrift verwendet werden, wobei der Klassenwert für alle Trainingsprodukte, die aus derselben Komponente bestehen, identisch ist, und
(3) die Abbildungsvorschrift in Form der Linearkombination aller oder ausgewählter Spektral- und/oder Summen-Spektralwerte bestimmt wird.

2. Verfahren nach Anspruch 1, **dadurch gekennzeichnet, dass** die wenigstens eine Abbildungsvorschrift einen Klassifikator darstellt, der die wenigstens zwei Komponenten vorgegebenen Klassen zuordnet.

3. Verfahren nach Anspruch 1 oder 2, **dadurch gekennzeichnet, dass** die Abbildungsvorschrift mit Hilfe multivariater Analyseverfahren, insbesondere der Korrelationsanalyse und der Diskriminanzanalyse bestimmt wird.

4. Verfahren nach Anspruch 2, **dadurch gekennzeichnet, dass** der Klassifikator ein künstliches neuronales Netz ist oder eine Support Vector Machine ist.

5. Verfahren nach einem der vorhergehenden Ansprüche, **dadurch gekennzeichnet, dass** die wenigstens eine Abbildungsvorschrift so bestimmt wird, dass aus den Bilddaten ein Dual-Energy-Bild oder Multiple-Energy-Bild erzeugt wird, indem die Spektralwerte von wenigstens zwei Gruppen von ausgewählten benachbarten Energiekanälen oder entsprechende Summen-Spektralwerte mit jeweils einem konstanten Gewichtsfaktor multipliziert werden und diese Produkte addiert werden.

6. Verfahren nach Anspruch 5, **dadurch gekennzeichnet, dass** die wenigstens eine Abbildungsvorschrift so bestimmt wird, dass sich die Gruppen von benachbarten Energiekanälen spektral nicht überlappen, oder dass der Röntgenstrahlungsdetektor so betrieben oder angesteuert wird, dass das Bildsignal nur Spektralwerte für sich nicht überlappende Gruppen von Energiekanälen beinhaltet oder bereits Summen-Spektralwerte für sich nicht überlappende Gruppen von Energiekanälen beinhaltet.

7. Verfahren nach einem der vorhergehenden Ansprüche, **dadurch gekennzeichnet, dass** anstelle von detektierten Spektralwerten oder Summen-Spektralwerten, die für ein Trainingsprodukt aus einer Komponente mit einem bekannten Material gewonnen werden, Simulationsdaten verwendet werden, wobei die Simulationsdaten Produkte aus vorbekannten Werten für den energieabhängigen Absorptionskoeffizienten und geeignet gewählten Dicken umfassen.

8. Verfahren nach einem der vorhergehenden Ansprüche, **dadurch gekennzeichnet, dass** mehrere Produkttypen definiert werden, wobei jeder Produkttyp Produkte umfasst, welche aus jeweils derselben ersten und wenigstens einer zweiten Komponente bestehen, die unterschiedliche Absorptionskoeffizienten für die Röntgenstrahlung aufweisen, und dass unter Verwendung derselben Bilddaten eines zu untersuchenden Produkts mehrere Gesamtbilder erzeugt werden, wobei zur Erzeugung jedes Gesamtbildes eine Abbildungsvorschrift für einen jeweils anderen Produkttyp verwendet wird.

9. Vorrichtung zur Röntgeninspektion von Produkten eines vorgegebenen Produkttyps, insbesondere von Lebensmitteln,

(a) mit einer Strahlungserzeugungsvorrichtung (104) mit mindestens einer Röntgenstrahlungsquelle (106) zur Erzeugung von Röntgenstrahlung mit einer vorgegebenen spektralen Breite,
(b) mit einem spektral auflösenden Röntgenstrahlungsdetektor (108), welcher eine vorgegebene Anzahl von Pixeln aufweist und die durch ein zu untersuchendes Produkt (102) hindurchgetretene Röntgenstrahlung an jedem Pixel diskret spektral aufgelöst detektiert, wobei der Röntgenstrahlungsdetektor (108) die Röntgenstrahlungsquanten zur spektralen Auflösung, abhängig von deren Energie, einer vorgegebenen Anzahl von Energiekanälen zuordnet und Bilddaten erzeugt, welche für jedes Pixel Spektralwerte für ausgewählte oder alle Energiekanäle und/oder Summen-Spektralwerte für eine oder mehrere Gruppen von benachbarten Energiekanälen beinhalten, und mit einer Auswerte- und Steuereinheit (132), welcher die von dem Röntgenstrahlungsdetektor (108) erzeugten Bilddaten zugeführt sind,

**dadurch gekennzeichnet,**

(c) **dass** die Auswerte- und Steuereinheit (132) zur Durchführung des Verfahrens nach einem der vorhergehenden Ansprüche ausgebildet ist.

10. Vorrichtung nach Anspruch 9, **dadurch gekennzeichnet, dass** die Auswerte- und Steuereinheit (132) einen Speicher oder Speicherbereich aufweist, in welchem die wenigstens eine Abbildungsvorschrift für wenigstens einen Produkttyp, insbesondere eine Vielzahl von Produkttypen, gespeichert ist.

**Claims**

1. Method for X-ray inspection of products of a predetermined product type, in particular food items,

(a) in which at least one product type is defined, which comprises products (102) which consist of at least a first and a second component which have different absorption coefficients for the X-ray radiation,

(b) in which a product (102) to be examined, which belongs to the at least one product type, is irradiated with X-ray radiation having a predetermined spectral width,

(c) in which the X-ray radiation passing through the product (102) is detected by means of a spectrally resolving X-ray radiation detector (200) which has a predetermined number of pixels and detects the X-ray radiation at each pixel in a discrete, spectrally resolved manner, the X-ray radiation detector (200) assigning the X-ray radiation quanta for spectral resolution, depending on their energy, to a predetermined number of energy channels and generating image data which contain spectral values for selected or all energy channels and/or sum spectral values for one or more groups of adjacent energy channels for each pixel, and

(d) in which at least one mapping rule is determined for processing the image data to form an overall image for the at least one product type, each mapping rule being designed such that all or selected spectral values and sum spectral values are mapped onto an overall image value of an image point of the overall image, a mapping rule being assigned to all pixels or predetermined groups of one or more pixels, and

(e) the one or more mapping rules being determined such that, in the overall image of a product (102) of the at least one product type, an improvement in the recognizability of product regions having different material properties is achieved compared to the use of non-spectrally resolving X-ray radiation detectors which only generate a simple gray scale image,

**characterized in that**

(f) the at least one mapping rule is determined as a linear combination by assigning a mapping coefficient to each spectral value or each sum spectral value, **and in that** the overall image of the product to be examined (102) is generated by multiplying each spectral value and each sum spectral value by the assigned imaging coefficient and adding these products, and

(g) **in that** the at least one imaging rule for the at least one product type is determined to improve the contrast by means of a machine learning method,

(iii) a plurality of training products, which each consist of one of the components and have different thicknesses, being detected in a learning mode by means of the spectrally resolving X-ray radiation detector (200),

(ii) to determine each mapping rule

(1) the spectral values or sum spectral values of all or selected pixels or groups of adjacent pixels detected for the training products being used as features of the mapping rule,

(2) predetermined class values being used as target values of the mapping rule, the class value being identical for all training products consisting of the same component, and

(3) the mapping rule being determined in the form of the linear combination of all or selected spectral and/or sum spectral values.

2. Method according to claim 1, **characterized in that** the at least one mapping rule represents a classifier which assigns the at least two components to predetermined classes.

3. Method according to either claim 1 or 2, **characterized in that** the mapping rule is determined using multivariate analysis methods, in particular correlation analysis and discriminant analysis.

**4.** Method according to claim 2, **characterized in that** the classifier is an artificial neural network or a support vector machine.

**5.** Method according to any one of the preceding claims, **characterized in that** the at least one imaging rule is determined such that a dual-energy image or multiple-energy image is generated from the image data by multiplying the spectral values of at least two groups of selected adjacent energy channels or corresponding sum spectral values by a constant weighting factor in each case and adding these products.

**6.** Method according to claim 5, **characterized in that** the at least one mapping rule is determined such that the groups of adjacent energy channels do not overlap spectrally, **or in that** the X-ray radiation detector is operated or controlled such that the image signal only contains spectral values for non-overlapping groups of energy channels or already contains sum spectral values for non-overlapping groups of energy channels.

**7.** Method according to any one of the preceding claims, **characterized in that** instead of detected spectral values or sum spectral values obtained for a training product from a component having a known material, simulation data are used, the simulation data comprising products of previously known values for the energy-dependent absorption coefficient and suitably selected thicknesses.

**8.** Method according to any one of the preceding claims, **characterized in that** a plurality of product types are defined, each product type comprising products which consist of the same first and at least one second component which have different absorption coefficients for X-ray radiation, **and in that** a plurality of overall images are generated using the same image data of a product to be examined, an imaging rule for a different product type being used to generate each overall image.

**9.** Device for X-ray inspection of products of a predetermined product type, in particular food items,

(a) having a radiation generating device (104) having at least one X-ray radiation source (106) for generating X-ray radiation having a predetermined spectral width,
(b) having a spectrally resolving X-ray radiation detector (108) which has a predetermined number of pixels and detects the X-ray radiation passing through a product (102) to be examined at each pixel in a discrete, spectrally resolved manner, the X-ray radiation detector (108) assigning the X-ray radiation quanta for spectral resolution, depending on their energy, to a predetermined number of energy channels and generating image data which contain spectral values for selected or all energy channels and/or sum spectral values for one or more groups of adjacent energy channels for each pixel, and having an evaluation and control unit (132) to which the image data generated by the X-ray radiation detector (108) are supplied,
**characterized in that**
(c) the evaluation and control unit (132) is designed to carry out the method according to any one of the preceding claims.

**10.** Device according to claim 9, **characterized in that** the evaluation and control unit (132) has a memory or memory region in which the at least one mapping rule for at least one product type, in particular a plurality of product types, is stored.

## Revendications

**1.** Procédé pour l'inspection par rayonnement X de produits d'un type de produit prédéfini, en particulier de produits alimentaires,

(a) dans lequel au moins un type de produit est défini, lequel comprend des produits (102) qui sont constitués d'au moins un premier et un second composant qui présentent des coefficients d'absorption différents pour le rayonnement X,
(b) dans lequel un produit (102) à examiner, lequel appartient à l'au moins un type de produit, est traversé par un rayon X comportant une largeur spectrale prédéfinie,
(c) dans lequel le rayonnement X qui a traversé le produit (102) est détecté au moyen d'un détecteur de rayonnement X (200) à résolution spectrale qui présente un nombre prédéfini de pixels et qui détecte le rayonnement X à une résolution spectrale discrète au niveau de chaque pixel, dans lequel le détecteur de rayonnement X (200) classe les quanta de rayonnement X pour la résolution spectrale, en fonction de leur énergie, en un nombre prédéfini de canaux d'énergie et génère des données d'image qui contiennent pour chaque pixel des valeurs spectrales pour une sélection ou pour tous les canaux d'énergie et/ou valeurs spectrales cumulées pour un ou plusieurs groupes de canaux d'énergie voisins, et

(d) dans lequel, pour le traitement des données d'image en une image globale pour l'au moins un type de produit, au moins une règle de représentation est déterminée, dans lequel chaque règle de représentation est réalisée de sorte qu'une sélection ou toutes les valeurs spectrales et valeurs spectrales cumulées sont représentées sur une valeur d'image globale d'un point d'image de l'image globale, dans lequel une règle de représentation est associée respectivement à tous les pixels ou à des groupes prédéterminés d'un ou de plusieurs pixels, et

(e) dans lequel la ou les règles de représentation sont déterminées de sorte que, dans l'image globale d'un produit (102) de l'au moins un type de produit, une amélioration de la détectabilité de zones de produit est atteinte, lesdites zones de produit comportant respectivement d'autres propriétés de matériau, par rapport à l'utilisation de détecteurs de rayonnement X à résolution non spectrale, lesquels ne génèrent qu'une simple image en niveaux de gris,

**caractérisé en ce que**

(f) l'au moins une règle de représentation est déterminée comme combinaison linéaire en associant un coefficient de représentation à chaque valeur spectrale ou à chaque valeur spectrale cumulée, **et en ce que** l'image globale du produit (102) à examiner est générée en multipliant chaque valeur spectrale et chaque valeur spectrale cumulée par le coefficient de représentation associé et en additionnant lesdits produits, et

(g) **que** l'au moins une règle de représentation pour l'au moins un type de produit est déterminée pour l'amélioration du contraste au moyen d'un procédé d'apprentissage automatique,

(i) dans lequel, dans un mode d'apprentissage, une pluralité de produits d'apprentissage, lesquels sont respectivement constitués de l'un des composants et présentent des épaisseurs différentes, sont détectés au moyen du détecteur de rayonnement X (200) à résolution spectrale,

(ii) dans lequel, pour la détermination de chaque règle de représentation

(1) les valeurs spectrales ou les valeurs spectrales cumulées d'une sélection ou de tous les pixels ou groupes de pixels adjacents, lesquels sont détectés pour les produits d'apprentissage, sont utilisées comme caractéristiques de la règle de représentation,

(2) des valeurs de classe prédéfinies sont utilisées comme valeurs cibles de la règle de représentation, dans lequel la valeur de classe est identique pour tous les produits d'apprentissage qui sont constitués du même composant, et

(3) la règle de représentation est déterminée sous forme de combinaison linéaire d'une sélection ou de toutes les valeurs spectrales et/ou valeurs spectrales cumulées.

2. Procédé selon la revendication 1, **caractérisé en ce que** l'au moins une règle de représentation représente un classificateur qui associe les au moins deux composants à des classes prédéfinies.

3. Procédé selon la revendication 1 ou 2, **caractérisé en ce que** la règle de représentation est déterminée à l'aide de procédés d'analyse multidimensionnels, en particulier l'analyse de corrélation et l'analyse discriminante.

4. Procédé selon la revendication 2, **caractérisé en ce que** le classificateur est un réseau neuronal artificiel ou est un séparateur à vaste marge.

5. Procédé selon l'une des revendications précédentes, **caractérisé en ce que** l'au moins une règle de représentation est déterminée de sorte qu'une image en double énergie ou une image en énergies multiples est générée à partir des données d'image en multipliant les valeurs spectrales d'au moins deux groupes de canaux d'énergie voisins sélectionnés ou les valeurs spectrales cumulées correspondantes par respectivement un facteur de pondération constant et en additionnant lesdits produits.

6. Procédé selon la revendication 5, **caractérisé en ce que** l'au moins une règle de représentation est déterminée de sorte que les groupes de canaux d'énergie voisins ne se chevauchent pas du point de vue spectral, **ou en ce que** le détecteur de rayonnement X fonctionne ou est commandé de sorte que le signal d'image ne contient que des valeurs spectrales pour des groupes de canaux d'énergie qui ne se chevauchent pas ou contient déjà des valeurs spectrales cumulées pour des groupes de canaux d'énergie qui ne se chevauchent pas.

7. Procédé selon l'une des revendications précédentes,

**caractérisé en ce que,** au lieu de valeurs spectrales détectées ou de valeurs spectrales cumulées obtenues pour un produit d'apprentissage à partir d'un composant comportant un matériau connu, des données de simulation sont utilisées, dans lequel les données de simulation comprennent des produits constitués de valeurs connues au préalable pour le coefficient d'absorption en fonction de l'énergie et des épaisseurs sélectionnées de manière appropriée.

8. Procédé selon l'une des revendications précédentes, **caractérisé en ce que** plusieurs types de produits sont définis, dans lequel chaque type de produit comprend des produits qui sont constitués respectivement du même premier composant et au moins un second composant qui présentent des coefficients d'absorption différents pour le rayonnement X, **et en ce que** plusieurs images globales sont générées en utilisant les mêmes données d'image d'un produit à examiner, dans lequel une règle de représentation est utilisée pour la génération de chaque image globale pour un type de produit respectivement différent.

9. Dispositif pour l'inspection par rayonnement X de produits d'un type de produit prédéfini, en particulier de produits alimentaires,

   (a) comportant un dispositif de génération de rayonnement (104) comportant au moins une source de rayonnement X (106) pour la génération d'un rayonnement X comportant une largeur spectrale prédéfinie,
   (b) comportant un détecteur de rayonnement X (108) à résolution spectrale qui présente un nombre prédéfini de pixels et qui détecte le rayonnement X à une résolution spectrale au niveau de chaque pixel, lequel rayonnement X a traversé un produit (102) à examiner, dans lequel le détecteur de rayonnement X (108) classe les quanta de rayonnement X pour la résolution spectrale, en fonction de leur énergie, en un nombre prédéfini de canaux d'énergie et génère des données d'image qui contiennent pour chaque pixel des valeurs spectrales pour une sélection ou pour tous les canaux d'énergie et/ou valeurs spectrales cumulées pour un ou plusieurs groupes de canaux d'énergie voisins, et comportant une unité d'évaluation et de commande (132) à laquelle sont amenées les données d'image générées par le détecteur de rayonnement X (108),
   **caractérisé en ce que**
   (c) l'unité d'évaluation et de commande (132) est réalisée pour la mise en oeuvre du procédé selon l'une des revendications précédentes.

10. Dispositif selon la revendication 9, **caractérisé en ce que** l'unité d'évaluation et de commande (132) présente une mémoire ou une zone de mémoire dans laquelle est enregistrée l'au moins une règle de représentation pour au moins un type de produit, en particulier une pluralité de types de produits.

**Fig. 1**

**Fig. 2**

**Fig. 3**

**Fig. 4**

Fig. 5a                    Fig. 5b                    Fig. 5c

| | Kanal1 | Kanal2 | Kanal3 | ... | Kanal256 | Klasse Y |
|---|---|---|---|---|---|---|
| Bild „Produkt" 1 | | | | | | Produkt |
| Bild „Produkt" 2 | | | | | | Produkt |
| Bild „Produkt" 3 | | | | | | Produkt |
| ... | | | | | | Produkt |
| Bild „Produkt" P | | | | | | Produkt |
| Bild „Kontamination" 1 | | | | | | Kontamination |
| Bild „Kontamination" 2 | | | | | | Kontamination |
| Bild „Kontamination" 3 | | | | | | Kontamination |
| ... | | | | | | Kontamination |
| Bild „Kontamination" K | | | | | | Kontamination |

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2588892 B1
- US 20180214113 A1
- US 20120213331 A1
- EP 2405260 A1